# EUROPÄISCHE PATENTANMELDUNG

(11) **EP 2 843 416 A1**
(43) Veröffentlichungstag der Anmeldung: **04.03.2015**
(21) Anmeldenummer: 13182563.0
(22) Anmeldetag: 02.09.2013
(51) Int. Cl.: G01N 33/86, G01N 33/96

(54) **Kontroll- und Kalibratormaterial für die Bestimmung von direkten Antikoagulanzien**

(71) Anmelder: Siemens Healthcare Diagnostics Products GmbH, 35041 Marburg (DE)
(72) Erfinder: Zander, Norbert, 35039 Marburg (DE); Pilgrim, Sabine, 35037 Marburg (DE); Wolf, Stefan, 35041 Marburg (DE)

(57) **Zusammenfassung**

Die Erfindung liegt auf dem Gebiet der Gerinnungsdiagnostik und betrifft ein Kontroll- und Kalibratormaterial enthaltend humanes und/oder tierisches Plasma sowie eine definierte Menge eines direkten Faktor IIa-Inhibitors, wie z.B. Hirudin, Dabigatran, Melagatran oder Argatroban, und eine definierte Menge eines direkten Faktor Xa-Inhibitors, wie z.B. Rivaroxaban, Apixaban oder Otamixaban.

## Beschreibung

Die vorliegende Erfindung liegt auf dem Gebiet der Gerinnungsdiagnostik und betrifft ein Kontroll- und Kalibratormaterial enthaltend humanes und/oder tierisches Plasma sowie eine definierte Menge eines direkten Faktor IIa-Inhibitors und eine definierte Menge eines direkten Faktor Xa-Inhibitors.

In der Antikoagulationstherapie werden zunehmend neue direkte Gerinnungsinhibitoren, insbesondere direkte Thrombin-(Faktor IIa-) und Faktor Xa-Inhibitoren eingesetzt. Diese neuen Gerinnungshemmer haben das Potenzial, die bislang benutzten indirekten Gerinnungshemmer, vor allem Heparin und dessen Derivate, die ihre gerinnungshemmende Wirkung nur im Zusammenspiel mit Kofaktoren wie Antithrombin oder Heparin Cofaktor II entfalten, abzulösen. Es ist daher notwendig über diagnostische Methoden zu verfügen, die es ermöglichen, die Plasmakonzentrationen der direkten Inhibitoren quantitativ zu bestimmen.

Im Stand der Technik sind verschiedene Testverfahren zum quantitativen Nachweis von direkten Faktor IIa- bzw. direkten Faktor Xa-Inhibitoren in humanen Plasmaproben bekannt.

Die Bestimmung von Faktor IIa- bzw. von Faktor Xa-Inhibitoren erfolgt überlicherweise mit Hilfe chromogener oder koagulometrischer Testverfahren. Bei den chromogenen Verfahren wird die Patientenprobe, die einen Faktor IIa- bzw. Faktor Xa-Inhibitor vermutlich enthält, mit einer sich im Überschuss befindlichen, definierten Menge des entsprechenden aktivierten Gerinnungsfaktors und einem chromogenen Substrat für den aktivierten Gerinnungsfaktor vermischt, und die im Reaktionsansatz verbleibende Aktivität des Gerinnungsfaktors wird photometrisch gemessen. Je höher die Konzentration des Inhibitors in der Patientenprobe ist, desto mehr wird die Aktivität des zugegebenen Gerinnungsfaktors gehemmt und desto geringer ist die gemessene Aktivität im Reaktionsansatz. Beispielsweise in EP-B1-0034320 oder in EP-A2-0004271 sind derartige Faktor IIa- bzw. Faktor Xa-basierte, chromogene Testverfahren beschrieben.

In EP-A1-2484776 ist ein Heparin-insensitives Verfahren für Faktor IIa- bzw. Faktor Xa-Inhibitoren beschrieben.

Eine zunehmende Zahl von direkten Faktor IIa- bzw. von Faktor Xa-Inhibitoren ist bereits für die therapeutische Anwendung zugelassen oder befindet sich in der Zulassung. Beispiele dafür sind die Substanzen Dabigatran (PRADAXA®), Argatroban (ARGATROBAN®), Rivaroxaban (XARELTO®), Apixaban (ELIQUIS®) und Edoxaban (LIXIANA®).

Für die Hersteller von diagnostischen Testen, die die quantitative Bestimmung der therapeutischen Faktor IIa- bzw. Faktor Xa-Inhibitoren in Patientenproben ermöglichen sollen, besteht das Problem, dass für jeden einzelnen Inhibitor spezifische Kontroll- und Kalibratormaterialien zur Verfügung gestellt werden müssen, die jeweils eine definierte Menge des jeweiligen Inhibitors enthalten. Üblicherweise wird als Kontroll- bzw. Kalibratormaterial normales Humanplasma verwendet, dem eine definierte Menge des jeweiligen Inhibitors hinzugefügt ist.

Auch für den Anwender ist die Vielzahl der benötigten Kontroll- und Kalibratormaterialien problematisch, da er die vielen verschiedenen Kontroll- und Kalibratormaterialien anschaffen und vorrätig halten muss. Insbesondere bei der Verwendung von automatischen Analysegeräten, die nur eine beschränkte Kapazität für die Aufnahme verschiedener Kontroll- und Kalibratormaterialien aufweisen, ist es praktisch nicht möglich, die für alle therapeutisch angewendeten Faktor IIa- und Faktor Xa-Inhibitoren spezifischen Kontroll- und Kalibratormaterialien im Gerät parallel zu platzieren. Ein weiterer Nachteil ist, dass die Haltbarkeit von Plasmakontrollen und -kalibratoren beschränkt ist, so dass Kontrollen und Kalibratoren für Teste, die weniger häufig durchgeführt werden, nicht vollständig aufgebraucht werden können bevor sie wegen Ablaufs des maximalen Haltbarkeit unverbraucht entsorgt werden müssen. Es ist daher wünschenswert, die Anzahl der notwendigen Kontroll-und Kalibratormaterialien zu reduzieren, um die vorgenannten Nachteile zu beseitigen und um die automatische Abarbeitung der verschiedenen spezifischen Teste auf einem Analysegerät sicherzustellen.

Der vorliegenden Erfindung lag daher die Aufgabe zugrunde, die geschilderten Probleme im Zusammenhang mit der Vielzahl der erforderlichen Plasmakontrollen und -kalibratoren zu beheben.

Die Aufgabe wird erfindungsgemäß dadurch gelöst, dass Plasmakontrollen bzw. -kalibratoren bereit gestellt werden, die gleichzeitig eine definierte Menge eines direkten Faktor IIa-Inhibitors und eine definierte Menge eines direkten Faktor Xa-Inhibitors enthalten.

Dies hat den Vorteil, dass die Anzahl der bereit zu stellenden Kontroll- bzw. Kalibratormaterialien für die quantitative Bestimmung von direkten Faktor IIa- und Faktor Xa-Inhibitoren um 50 % reduziert wird.

Gegenstand der vorliegenden Erfindung ist eine Zubereitung enthaltend humanes und/oder tierisches Plasma und eine definierte Menge eines direkten Faktor IIa-Inhibitors und eine definierte Menge eines direkten Faktor Xa-Inhibitors.

Die Herstellung einer erfindungsgemäßen Zubereitung erfolgt üblicherweise, indem einem humanem Normalplasma, einem tierischen Plasma, z.B. aus Rind oder Kaninchen oder einer Mischung aus humanem und tierischem Plasma eine definierte Menge eines direkten Faktor IIa-Inhibitors und eine definierte Menge eines direkten Faktor Xa-Inhibitors zugegeben wird.

Der Begriff "direkter Faktor IIa-Inhibitor" bezeichnet therapeutisch wirksame Substanzen, die natürlicherweise nicht im menschlichen Körper vorkommen und die durch direkte Interaktion mit Faktor IIa (Thrombin) die Aktivität von Faktor IIa inhibieren. Direkte Faktor IIa-Inhibitoren sind zu unterscheiden von "indirekten Inhibitoren", wie z.B. Heparin, welche nur durch die Interaktion mit physiologischen Kofaktoren, wie z.B. Antithrombin oder Heparin Cofaktor II, die Aktivität von Faktor IIa indirekt reduzieren. Der Begriff "direkter Faktor IIa-Inhibitor" umfasst explizit nicht Substanzen, die natürlicherweise im menschlichen Körper vorkommen und die durch direkte Interaktion mit Faktor IIa (Thrombin) die Aktivität von Faktor IIa inhibieren, wie z.B. Antithrombin oder Heparin Cofaktor II.

Geeignete direkte Faktor IIa-Inhibitoren sind beispielsweise Hirudin, Dabigatran, Melagatran, Argatroban, Ximelagatran, Bivalirudin, Lepirudin, MCC-977, SSR-182289, TGN-255, TGN-167, ARC-183 und Odiparcil.

Eine bevorzugte Zubereitung enthält 10-1000 ng/mL Dabigatran oder 100-3000 ng/mL Argatroban oder 100-3000 ng/mL Hirudin.

Der Begriff "direkter Faktor Xa-Inhibitor" bezeichnet therapeutisch wirksame Substanzen, die natürlicherweise nicht im menschlichen Körper vorkommen und die durch direkte Interaktion mit Faktor Xa die Aktivität von Faktor Xa inhibieren. Direkte Faktor Xa-Inhibitoren sind zu unterscheiden von "indirekten Inhibitoren", wie z.B. Heparin, welche nur durch die Interaktion mit physiologischen Kofaktoren, wie z.B. Antithrombin oder Heparin Cofaktor II, die Aktivität von Faktor Xa indirekt reduzieren. Der Begriff "direkter Faktor Xa-Inhibitor" umfasst explizit nicht Substanzen, die natürlicherweise im menschlichen Körper vorkommen und die durch direkte Interaktion mit Faktor Xa die Aktivität von Faktor Xa inhibieren, wie z.B. Antithrombin oder Heparin Cofaktor II.

Geeignete direkte Faktor Xa-Inhibitoren sind beispielsweise Rivaroxaban, Apixaban, Edoxaban, Otamixaban (welche in der neuen Wirkstoffklasse der Xabane zusammengefasst werden),LY 517717, YM 153, DU-176b, DX-9065a und KFA-1982.

Eine bevorzugte Zubereitung enthält 10-1000 ng/mL Rivaroxaban oder 10-1000 ng/mL Apixaban.

In einer bevorzugten Ausführungsform enthält die Zubereitung humanes Normalplasma und eine definierte Menge des direkten Faktor IIa-Inhibitors Dabigatran und eine definierte Menge des direkten Faktor Xa-Inhibitors Rivaroxaban.

In einer anderen bevorzugten Ausführungsform enthält die Zubereitung humanes Normalplasma und eine definierte Menge des direkten Faktor IIa-Inhibitors Dabigatran und eine definierte Menge des direkten Faktor Xa-Inhibitors Apixaban.

Eine erfindungsgemäße Zubereitung kann in flüssiger Form oder als Lyophilisat bereit gestellt werden.

Ein weiterer Gegenstand der vorliegenden Erfindung ist die Verwendung einer erfindungsgemäßen Zubereitung als Kontroll-oder Kalibratormaterial in einem Verfahren zur quantitativen Bestimmung eines direkten Faktor IIa-Inhibitors oder in einem Verfahren zur quantitativen Bestimmung eines direkten Faktor Xa-Inhibitors in einer Probe.

Unter einer "Probe" ist im Sinne der Erfindung das Material zu verstehen, das den zu bestimmenden direkten Gerinnungsinhibitor vermutlich enthält. Der Begriff Probe umfasst insbesondere menschliche oder tierische Körperflüssigkeiten, vornehmlich Blut, Plasma und Serum.

Die Probe kann beispielsweise einem konventionellen Gerinnungsfaktoraktivitätstest unterworfen werden, wobei der Probe eine definierte Menge desjenigen aktivierten Gerinnungsfaktors zugegeben wird, für welchen der zu bestimmende direkte Gerinnungsinhibitor spezifisch ist. Bei einem Verfahren zur Bestimmung eines direkten Faktor IIa-Inhibitors wird konsequenterweise eine definierte Menge Thrombin zugegeben; bei einem Verfahren zur Bestimmung eines direkten Faktor Xa-Inhibitors wird eine definierte Menge Faktor Xa zugegeben, und die Inhibitor-abhängige Inaktivierung der Aktivität des zugegebenen Gerinnungsfaktors wird gemessen. Bevorzugterweise wird die Inhibitor-abhängige Inaktivierung der amidolytischen Aktivität des zugegebenen Gerinnungsfaktors mit Hilfe eines chromogenen, fluorogenen oder anderweitig markierten Substrats ermittelt, das spezifisch von dem aktivierten Gerinnungsfaktor gespalten wird. Bei der abspaltbaren Signalgruppe eines Substrats kann es sich z.B. um einen im sichtbaren Bereich des Spektrums bestimmbaren Farbstoff, einen Fluoreszenzfarbstoff oder einen im UV-Bereich bestimmbaren Farbstoff handeln. Bevorzugterweise werden Peptide verwendet, die an der Carboxygruppe eines Argininrests einen durch Amidbindung gebundenen Farbstoffrest aufweisen. Besonders geeignet sind hierfür p-Nitroanilidgruppen (pNA) und 5-Amino-2-Nitro-Benzoesäurederivate (ANBA), sowie von diesen durch Substitution abgeleitete Farbstoffe, welche nach Abspaltung vom Peptidanteil durch eine photometrische Messung bei 405 nm Wellenlänge quantifiziert werden können. Bevorzugterweise besteht der Peptidanteil eines spaltbaren Substrats aus 3 bis etwa 150 Aminosäureresten. In den Patentdokumenten EP-A1-0034122 und US 4,508,644 sind eine Vielzahl von geeigneten chromogenen Peptidsubstraten, deren Herstellung und deren Verwendung in gerinnungsdiagnostischen Tests beschrieben, z.B. zur Bestimmung der Gerinnungsfaktoren Faktor IIa (Thrombin) und Xa.

Die Menge des freigesetzten signalbildenden Spaltprodukts, z.B. der chromogenen, fluorogenen oder amperogenen Gruppe, verhält sich umgekehrt proportional zur Inhibitoraktivität bzw. -konzentration in der Probe. Mit Hilfe einer Kalibrationskurve, erstellt aus der Messung von erfindungsgemäßen Zubereitungen mit bekannten Inhibitoraktivitäten, lässt sich die Menge eines direkten Gerinnungsinhibitors in einer Patientenprobe korrekt quantifizieren.

Die amidolytische Aktivität kann kinetisch oder als Endpunktbestimmung ausgewertet werden. Bei der kinetischen Methode wird die Reaktion, d.h. die verbliebene Gerinnungsfaktoraktivität als Maß für die vorhandene Inhibitoraktivität, anhand der Umsatzrate des spaltbaren Substrats quantifiziert. Bei der Endpunktbestimmung wird nach einer vorbestimmten Messzeit die Spaltreaktion gestoppt und die Menge des freigesetzten Spaltprodukts gemessen.

Ein weiterer Gegenstand der vorliegenden Erfindung ist ein Testkit enthaltend ein erstes Reagenz enthaltend eine definierte Menge Thrombin, ein zweites Reagenz enthaltend eine definierte Menge Faktor Xa, ein drittes Reagenz enthaltend ein Thrombin-spezifisches Substrat, ein viertes Reagenz enthaltend ein Faktor Xa-spezifisches Substrat sowie eine erfindungsgemäße Zubereitung enthaltend humanes und/oder tierisches Plasma und eine definierte Menge eines direkten Faktor IIa-Inhibitors und eine definierte Menge eines direkten Faktor Xa-Inhibitors.

In einer bevorzugten Ausführungsform eines Testkits enthält die erfindungsgemäße Zubereitung eine definierte Menge des direkten Faktor IIa-Inhibitors Dabigatran und eine definierte Menge des direkten Faktor Xa-Inhibitors Rivaroxaban.

In einer anderen bevorzugten Ausführungsform eines Testkits enthält die erfindungsgemäße Zubereitung eine definierte Menge des direkten Faktor IIa-Inhibitors Dabigatran und eine definierte Menge des direkten Faktor Xa-Inhibitors Apixaban.

### Figurenbeschreibung

**Figur 1**
   zeigt Kalibrationskurven für einen chromogenen Faktor Xa-Test mit einem Kalibrator enthaltend nur Rivaroxaban (Kurve 1), mit einem Kalibrator enthaltend Rivaroxaban und Dabigatran (Kurve 2) und mit einem Kalibrator enthaltend nur Dabigatran (Kurve 3).
**Figur 2**
   zeigt Kalibrationskurven für einen chromogenen Faktor IIa-Test mit einem Kalibrator enthaltend nur Rivaroxaban (Kurve 1), mit einem Kalibrator enthaltend Rivaroxaban und Dabigatran (Kurve 2) und mit einem Kalibrator enthaltend nur Dabigatran (Kurve 3).

### BEISPIELE

### Beispiel 1 : Herstellung eines erfindungsgemäßen Dabigatran-Rivaroxaban-Kalibrators

Zur Herstellung eines erfindungsgemäßen Dabigatran-Rivaroxaban-Kalibrators wurden einem normalen, humanen Citratplasmapool (Kontrol Plasma N, Siemens Healthcare Diagnostics, Marburg) 500 ng/mL Dabigatran (Selleckchem) und 500 ng/mL Rivaroxaban (Selleckchem) hinzugegeben.

Desweiteren wurden als Kontrollpräparationen ein Citratplasmapool mit 500 ng/mL Dabigatran und ein weiterer Citratplasmapool mit 500 ng/mL Rivaroxaban hergestellt.

### Beispiel 2: Kalibration eines Faktor Xa-Testes

Die drei Präparationen wurden auf einem Gerinnungsmessgerät (BCS® XP, Siemens Healthcare Diagnostics, Marburg) als Kalibratoren in einem chromogenen, kompetitiven Test eingesetzt, mit dem die Quantifizierung von direkten Faktor Xa-Inhibitoren möglich ist. Das Gerinnungsmessgerät verdünnt dabei die Präparationen automatisch auf vordefinierte, niedrigere Konzentrationen, um so eine Kalibrationskurve zu erzeugen.

Der Test besteht aus einem Reagenz I mit einer definierten Konzentration Faktor Xa, einem Reagenz II mit einer definierten Konzentration eines durch Faktor Xa hydrolysierbaren Substrates (Z-D-Leu-Gly-Arg-ANBA-methylamid) und einem Reagenz III, welches ein Verdünnungsmedium darstellt. In den Reaktionsansatz wird die mit Reagenz III verdünnte Probe zusammen mit Reagenz I und Reagenz II vermischt und die Geschwindigkeit des Anstiegs der Absorption bei 405 nm gemessen, welche ein Maß für die Substratspaltung durch Faktor Xa darstellt.

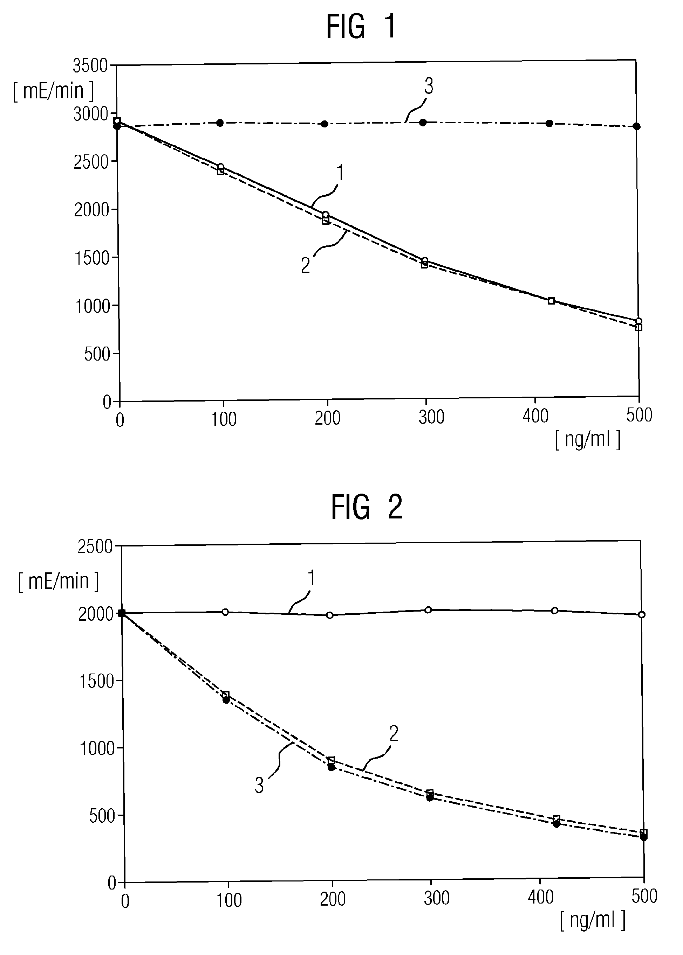

Figur 1 zeigt, dass die Präparation, welche gleichzeitig Dabigatran und Rivaroxaban enthält, eine vergleichbare Kalibrationskurve erzeugt wie die Präparation, welche nur Rivaroxaban enthält und ist somit gleichermaßen für eine Kalibration in dieser Testmethode geeignet.

### Beispiel 3: Kalibration eines Faktor IIa-Testes

Desweiteren wurden die drei Präparationen auf einem Gerinnungsmessgerät (BCS® XP, Siemens Healthcare Diagnostics, Marburg) als Kalibrator in einem chromogenen, kompetitiven Test zur Quantifizierung von direkten Faktor IIa-Inhibitoren eingesetzt.

Das Gerinnungsmessgerät verdünnt dabei die Präparationen automatisch mit einem normalen humanen Citratplasma ohne Faktor IIa-Inhibitor auf vordefinierte, niedrigere Konzentrationen, um so eine Kalibrationskurve zu erzeugen. Der Test besteht aus einem Reagenz I mit einer definierten Konzentration Faktor IIa und einem Reagenz II mit einer definierten Konzentration eines durch Faktor IIa hydrolysierbaren Substrates (Tos-Gly-Pro-Arg-ANBA-Isopropylamid). Die Probe wird mit Reagenz I und Reagenz II vermischt, und die Geschwindigkeit des Anstiegs der Absorption wird bei 405 nm gemessen, welche ein Maß für die Substratspaltung durch Faktor IIa darstellt.

Figur 2 zeigt, dass die Präparation, welche gleichzeitig Dabigatran und Rivaroxaban enthält, eine vergleichbare Kalibrationskurve erzeugt wie die Präparation, welche nur Dabigatran enthält und ist somit gleichermaßen für eine Kalibration in dieser Testmethode geeignet.

## Patentansprüche

1. Zubereitung enthaltend humanes und/oder tierisches Plasma und eine definierte Menge eines direkten Faktor IIa-Inhibitors und eine definierte Menge eines direkten Faktor Xa-Inhibitors.

2. Zubereitung nach Anspruch 1, enthaltend einen direkten Faktor IIa-Inhibitor aus der Gruppe Hirudin, Dabigatran, Melagatran, Argatroban, Ximelagatran, Bivalirudin, Lepirudin, MCC-977, SSR-182289, TGN-255, TGN-167, ARC-183 und Odiparcil.

3. Zubereitung nach einem der vorhergehenden Ansprüche, enthaltend einen direkten Faktor Xa-Inhibitor aus der Gruppe Rivaroxaban, Apixaban, Otamixaban, LY 517717, YM 153, DU-176b, DX-9065a und KFA-1982.

4. Zubereitung nach einem der vorhergehenden Ansprüche, enthaltend Dabigatran und Rivaroxaban.

5. Zubereitung nach einem der vorhergehenden Ansprüche, enthaltend Dabigatran und Apixaban.

6. Zubereitung nach einem der vorhergehenden Ansprüche, enthaltend 10-1000 ng/mL Dabigatran.

7. Zubereitung nach einem der vorhergehenden Ansprüche, enthaltend 10-1000 ng/mL Rivaroxaban oder Apixaban.

8. Verwendung einer Zubereitung gemäß einem der Ansprüche 1-7 als Kontroll- oder Kalibratormaterial in einem Verfahren zur quantitativen Bestimmung eines direkten Faktor IIa-Inhibitors in einer Probe.

9. Verwendung einer Zubereitung gemäß einem der Ansprüche 1-7 als Kontroll- oder Kalibratormaterial in einem Verfahren zur quantitativen Bestimmung eines direkten Faktor Xa-Inhibitors in einer Probe.

10. Testkit enthaltend
a. ein erstes Reagenz enthaltend eine definierte Menge Thrombin,
b. ein zweites Reagenz enthaltend eine definierte Menge Faktor Xa,
c. ein drittes Reagenz enthaltend ein Thrombin-spezifisches Substrat und ein Faktor Xa-spezifisches Substrat, oder
d. ein drittes Reagenz enthaltend ein Thrombin-spezifisches Substrat und ein viertes Reagenz enthaltend ein Faktor Xa-spezifisches Substrat sowie
e. eine Zubereitung gemäß einem der Ansprüche 1-7.
